# EUROPEAN PATENT APPLICATION

(11) **EP 4 796 028 A1**
(43) Date of publication of application: **26.08.2026**
(21) Application number: 25159345.5
(22) Date of filing: 21.02.2025
(51) Int. Cl.: A61B 5/11, A61B 5/00

(54) **DEVICE FOR MEASURING DORSAL MOBILITY OF A FOOT**

(71) Applicant: Schoinas, Spyridon, 1206 Geneva (CH); Praz, Quentin, 01280 Prévessin-Möens (FR)
(72) Inventor: Schoinas, Spyridon, 1206 Geneva (CH); Praz, Quentin, 01280 Prévessin-Möens (FR)
(74) Representative: KATZAROV S.A.

(57) **Abstract**

The present invention relates to a device for measuring the dorsal mobility of the first ray of the foot comprising two independent upward force exerting means (2) adapted to exert a force on a part of a forefoot, each upward force exerting means (2) comprises a force sensor, wherein the device comprises at least one displacement sensor (5), the device is adapted to measure a displacement as a function of the applied force.

## Description

### Technical Field

The present invention relates to a device for measuring the dorsal mobility of a foot.

### Background

The term hypermobility or instability of the first ray is used to describe an excessive range of motion of the joints related to the first ray of the foot beyond what is considered "normal". The first ray hypermobility is associated with the development of several forefoot disorders such as hallux valgus, metatarsalgia, stress fractures, flatfoot, Lisfranc chronic dislocation or dislocation of the second metatarsophalangeal joint. The biomechanical function of the first ray plays an important role in the dynamic balance of the foot, particularly by preparing the propulsive phase of the gait, thus leading to a high number of different surgical interventions when instability and other symptoms occur. However, clinical assessment of first ray mobility is mainly empirical for clinicians.

EP 4 518 752 describes an instrument developed to measure the relative dorsal mobility of the first ray. This device equally applies a manually controlled force under the first metatarsal head M1 as well as under the heads of the lesser metatarsals M2 to M5. The relative dorsal mobility between these two bearings is then measured.

However, there is a need for a device that can measure the absolute dorsal mobility of the first ray as a function of an electronically controlled applied force under the M1 and M2-M5 metatarsal heads, while simulating partial weight support in a standing position to replicate ground reaction force.

The present invention thus suggests a device that permits measuring the absolute dorsal mobility of the first ray, by applying independent forces under the M1 and M2-M5 metatarsal heads, to improve diagnostics pursued by practitioners.

### Summary

The present invention is directed to a device for measuring the dorsal mobility of the first ray of the foot comprising two independent upward force exerting means adapted to exert a force on a part of a forefoot, each upward force exerting means comprises a force sensor, wherein the device comprises at least one displacement sensor, the device is adapted to measure a displacement as a function of the applied force.

According to an embodiment, each force exerting means comprises a displacement sensor.

According to one characteristic, the two upward force exerting means are arranged in opposition to each other and are adapted to exert a force respectively on the internal and external part of the forefoot.

According to a further characteristic, each upward force exerting means comprises an output shaft driven by an actuator.

According to the previous characteristic, the actuator is an electronically controlled actuator.

The actuator is advantageously a stepper motor associated with a digital stepper driver as a displacement sensor.

According to a supplementary characteristic, the output shaft is perpendicularly connected to a support arm mobile in translation along a linear guide directed upwardly.

According to the previous characteristic, the support arm is connected to a lead screw coupled to the actuator, while the lead screw is arranged parallel to the linear guide.

The support arm comprises preferentially a first arm connected to the output shaft, a linear carriage mounted along the linear guide, and second arm mounted along the lead screw.

According to another characteristic, the distal end of the output shaft is linked to a forefoot support adapted to support sensibly half of the lateral part of the forefoot.

According to the previous characteristic, the forefoot support comprises a matrix array sensor.

According to one implementation, the two independent upward force exerting means are laterally mobile in translation to adapt to the width of a forefoot.

According to the previous implementation, each upward force exerting means are independently, laterally mobile in translation.

Each upward force exerting means is advantageously disposed on a sliding platform driven by a secondary actuator.

According to a characteristic, the device comprises a hindfoot blocking support.

### Brief description of the drawings

Further particular advantages and features of the invention will become more apparent from the following non-limitative description of at least one embodiment of the invention which will refer to the accompanying drawings, wherein

Figures 1 to 7 illustrate the device according to an embodiment of the invention:
- Figure 1 is a top view showing the positioning of a foot on the device.
- Figure 2 is a top view without the foot and without the housing on the top of the two upward force exerting means, showing the top part of the two upward force exerting means.
- Figure 3 is an elevated view of figure 2.
- Figure 4 is a side view of figure 3 with a foot.
- Figure 5 is a fully elevated view of the two upward force exerting means mounted on a sliding platform.
- Figure 6 is a top view of figure 5.
- Figure 7 is a cross-sectional view of figure 6 taken along the lines B-B.
- Figure 8 is a graph showing measurements of the absolute displacement as a function of the applied force of the two actuators on the two force sensors.
- Figure 9 is a graph showing measurements of the relative displacement between the two actuators as a function of the sum of the total applied force exerted on both force sensors.
- Figure 10 is a graph showing the control of the applied force of an actuator in function of time.

### Detailed description

The device (1) according to the invention is intended to exert two electronically controlled upward forces on the two lateral parts of the forefoot, more precisely at the junction of the proximal part of the forefoot and the distal part of the midfoot of a patient, i.e., the inside part and the outside part of the group of metatarsal heads forming the distal part of the midfoot, advantageously configured for bearing against the first metatarsal head M1 and the lesser metatarsal heads M2 to M5 of the forefoot.

The device (1) is thus intended to measure the dorsal mobility of the first ray by measuring the absolute and/or relative displacement of the two lateral parts of the forefoot of a patient, more precisely at the level of the metatarsal heads.

More precisely, it is intended by controlled upward forces, equal forces or different forces on the M1 and M2-M5 metatarsal heads respectively, and the device (1) is thus able to measure independently absolute displacement of the M1 and M2-M5 metatarsal heads, as well as relative displacement between the M1 and M2-M5 metatarsal heads.

Applying and measuring forces against the first metatarsal head M1 and the lesser metatarsal heads M2 to M5 of the forefoot permit to assess the dorsal mobility of the first ray, i.e., the cuneiform bones which are a set of three bones in the medial side of the foot that articulate with the navicular proximally and with the proximal surfaces of metatarsal M1 to M3 distally.

It should be understood that the metatarsal bones are a group of five long bones in the midfoot, located between the tarsal bones and the phalanges. The metatarsal bones are numbered from the medial side, the side of the great toe, i.e., the first M1, second M2, third M3, fourth M4, and fifth M5 metatarsals.

Applying equal forces on M1 and M2-M5 metatarsal heads allow to simulate the ground reaction force applied under the foot.

Applying different forces on M1 and M2-M5 metatarsal heads permits to measure the absolute or relative mobility of the first ray of the foot under different conditions in order to reproduce other techniques described in the literature, such as Morton's technique, which consists of applying a dorsal force under the first metatarsal head with one hand, while the lower metatarsal heads are held fixed with the other hand.

It should also be understood that an absolute value of displacement corresponds to how far an object moves regardless of the direction and is equal to the absolute value, or magnitude, of the displacement, while a relative value of displacement corresponds to the relative value between two objects, i.e., in the present case the difference of displacement between the first metatarsal head M1 and the lesser metatarsal heads M2 to M5 of the forefoot, and is equal to the difference of absolute displacements of the first metatarsal head M1 and the lesser metatarsal heads M2 to M5.

The dorsal mobility of the first ray is the range of motion of the joints related to the first ray of the foot, when it is displaced in the dorsal direction..

The device (1) comprises two independent upward force exerting means (2), each equipped with a force sensor, while the device (1) comprises at least one displacement sensor (5), the device (1) is adapted to measure a displacement as a function of the applied force.

More precisely, the two upward force exerting means (2) are independent in terms of applied forces by independent actuators (6) as explained in more detail later in the description, but are connected electronically for correlating measurements, via microcontrollers and associated computer.

According to the illustrated embodiment, each independent upward force exerting means (2) comprises a force sensor and a displacement sensor (5).

According to another embodiment not illustrated, each independent upward force exerting means (2) comprises a force sensor, while the device comprises a displacement sensor (5) such as a camera, or two optical sensors to measure the displacement of the two upward force exerting means (2).

The two upward force exerting means (2) are intended to exert an electronically controlled upward force on two lateral parts of the forefoot of a patient, more precisely at the level of the metatarsal heads.

The two upward force exerting means (2) are located in opposition to each other.

According to an embodiment, the two independent upward force exerting means (2), each comprises one independent output shaft (3) to exert an upward force on a part of the patient's foot, each output shaft (3) is connected to a force sensor and advantageous to a displacement sensor (5), to provide a relative or absolute displacement as a function of the applied force.

The two output shafts (3) are arranged in parallel to each other, to exert two upward forces to two lateral parts of the forefoot of a patient.

According to the illustrated embodiment, the upward force exerting means (2) comprises an output shaft (3) linked to a force sensor, driven upward by an actuator (6) comprising or associated with a displacement sensor (5).

The actuator (6) is preferably an electronically controlled actuator (6) as a DC motor or a stepper motor, preferably a stepper motor.

The displacement sensor (5) associated with the actuator is chosen among an optical encoder, a mechanical encoder, a graduated scale, a laser sensor, an electromagnetic sensor for instance a linear variable differential transformer, or a digital stepper driver.

According to the illustrated embodiment, the actuator (6) is a stepper motor associated with a digital stepper driver as a displacement sensor (5).

According to one characteristic, the force sensor is chosen among a load cell, a compression load cell or a spring dynamometer, preferably a load cell.

According to the illustrated embodiment, the device (1) comprises two independent parallel output shafts (3), the proximal end of each output shaft (3) being connected to a force sensor and to a support arm (7) guided along a linear guide (8), each support arm (7) is connected to a displacement sensor (5) and is driven by an actuator (6) to drive each output shaft (3) parallel to the corresponding linear guide (8).

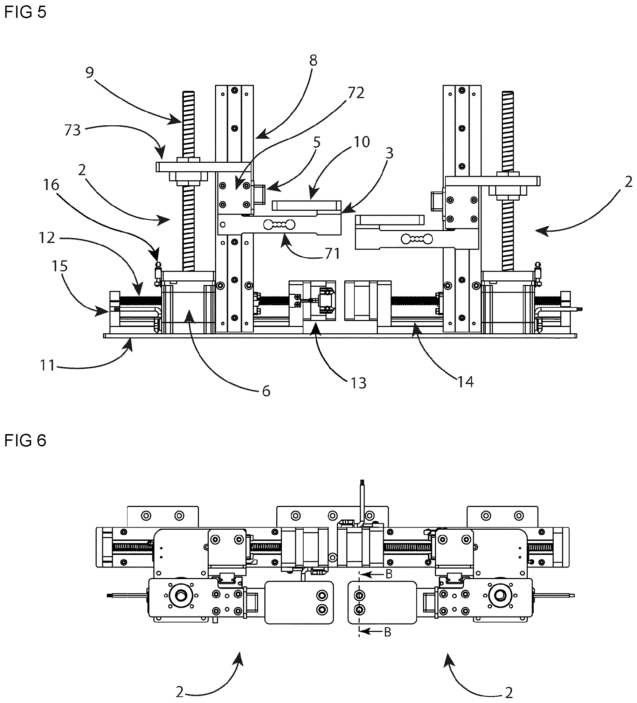

As illustrated in figure 5, an upward force exerting means (2) comprises a linear guide (8) directed upwardly, on which is mounted a linear carriage (72) adapted to move up and down along the linear guide (8).

According to the illustrated embodiment, the support arm (7) comprises a first arm (71), the carriage (72) and a second arm (73).

The linear carriage (72) is coupled to a first arm (71) and to a second arm (73), while the first arm (71) and the second arm (73) are parallel to each other and orthogonal to the linear guide (8).

More precisely the proximal end of the first arm (71) and the proximal end of the second arm (73) are connected to the carriage (72).

The first arm (71) and the second arm (73) are directed in opposite directions to each other compared to the carriage (72).

The first arm (71) comprises the force sensor, preferably a load cell within its housing.

The distal end or sensibly the distal end of the first arm (71) is connected perpendicularly to the output shaft (3).

The output shaft (3) materialized in the illustrated embodiment corresponds to the junction of the first arm (71) and the foot support (10), i.e., the junction in which the applied force is transmitted from the first arm (71) to the foot support (10) as explained later in the description. The latter mentioned junction as an output shaft (3) is perpendicular to the first arm (71) and the foot support (10).

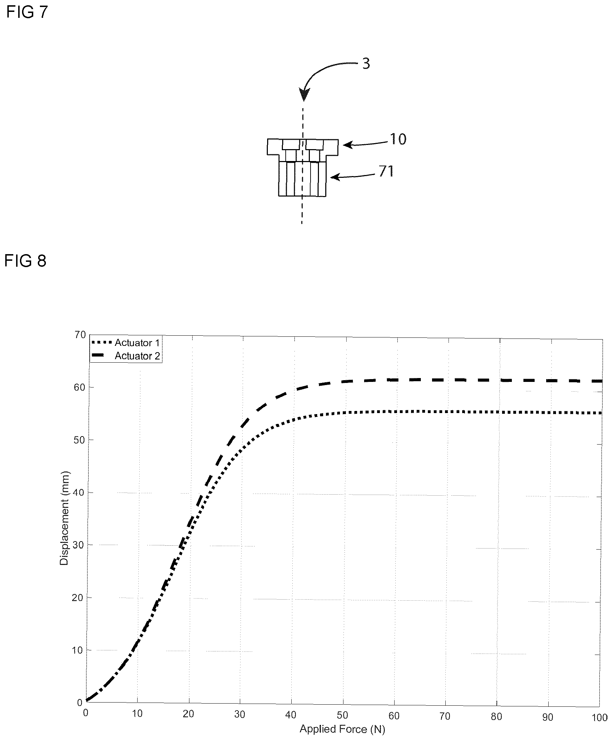

More precisely, the output shaft (3) is represented by the upward oriented axis located between the two screws joining the first arm (71) and the foot support (10), as illustrated in figure 7. In other words, the output shaft (3) is represented by the distal end of the first arm (71) and the proximal end of foot support (10).

The second arm (73) is mounted in translation along a lead screw (9), while the lead screw (9) is disposed parallel to the linear guide (8).

The lead screw (9) is connected to the actuator (6), while the functioning of the actuator (6) turns the lead screw (9) to move in translation the second arm (73) which drives the carriage (72) along the linear guide (8), and moves dependent of the direction of rotation of the lead screw (9), the first arm (71) up and down.

The actuator (6) is advantageous fixed to the linear guide (8).

According to another characteristic, an upward force exerting means (2) is mobile in lateral translation to adapt the position of the output shaft (3) to the width of the forefoot of the patient.

More precisely, according to the illustrated embodiment, the linear guide (8) and the actuator (6) are mounted on a sliding platform (11), while the sliding platform (11) is disposed perpendicularly to the linear guide (8).

A secondary screw (12) is embedded within the housing of the linear guide (8) and the actuator (6) by threated connection. The secondary screw (12) is connected to a secondary actuator (13), such as a stepper motor, whose operation leads to the translation of the linear guide (8) and the actuator (6) along the sliding platform (11).

The secondary screw (12) is arranged orthogonally to the lead screw (9).

To optimize space requirements, the secondary actuator (13) is positioned in opposition to the actuator (6) compared to the linear guide (8), while the actuator (6) is located on the exterior side of the linear guide (8) and the secondary actuator (13) is arranged on the interior of the linear guide (8).

As the two upward force exerting means (2) are arranged in opposition, the interior of the linear guide (8) corresponds to the direction between the two upward force exerting means (2).

According to the illustrated embodiment, the sliding platform (11) supports the two independent upward force exerting means (2).

More precisely, the sliding platform (11) comprises two coaxial rails (14) with advantageously sliding channels to support corresponding linear guide (8) and actuator (6). The linear guide (8) and the actuator (6) thus comprise counter form to the sliding channel of the rail (14), such as groove.

Each rail (14) comprises at its external end a rail stop (15) and a secondary actuator (13) at its internal end. The secondary screw (12) is held by the rail stop (15) and the secondary actuator (13).

As illustrated in figure 5, the actuator (6) comprises a vertical and a horizontal limit switches (16), for limiting the position of the upward force exerting means (2) along the lead screw (9) and the secondary screw (12) respectively.

According to a supplementary characteristic, the upward force exerting means (2) comprises a foot support (10) disposed at the distal end of the output shaft (3).

The foot support (10) is elongated and arranged parallel to the first arm (71), while the foot support (10) is directed in the opposite direction of the first arm (71), i.e. the distal end of the foot support (10) is directed to the proximal end of the first arm (71).

The applied force by the actuator (6) is thus transmitted to the second leg (73) to the carriage (72) and to the first leg (71), and finally to the foot support (10) by the output shaft (3).

According to another characteristic, the upward force exerting means (2) comprises a positioning sensor (5) of the forefoot resting against the output shaft (3), more precisely on the foot support (10).

More precisely the two positioning sensors (5) arranged on each opposite upward force exerting means (2) permit to measure and determine the position of the forefoot on the two foot supports (10).

Adaptation of the width between the two upward force exerting means (2) by the secondary actuator (13), allows to adapt to the forefoot width to exert upward force on the right location under the foot, i.e., against the first metatarsal head M1 by one output shaft (3) via corresponding foot support (10) and against the lesser metatarsal heads M2 to M5 by the second output shaft (3) via corresponding foot support (10).

According to figure 5, the positioning sensor (5) is located on the carriage (72), more precisely located on the interior side of the carriage (72) to face the forefoot.

The positioning sensor (5) is preferentially an optical sensor.

According to one implementation, the foot support (10) comprises a matrix array sensor, not shown.

It is understood that a matrix array sensor comprises piezoresistive sensors arranged in matrix form whose resistance varies depending on the force/pressure applied.

The measure by the matrix array sensors, of the load transfer of a foot along the metatarsal heads by the foot supports (10) while exerting an upward force, will help the surgeon to diagnose excessive pressure on the five metatarsal heads and adjust the surgical correction.

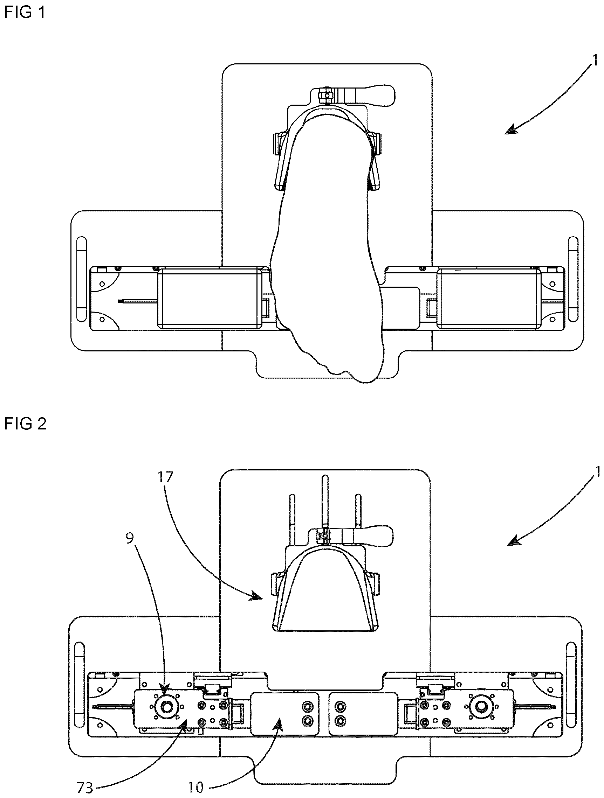

According to figure 1, 2 and 4, the device (1) comprises a hindfoot blocking support (17) located on the device's housing, to block the first ray from moving when upward forces are applied to the forefoot by the two upward force exerting means (2).

The hindfoot blocking support (17) is mounted on sliding guide with an adjusting switch to adapt its position to the length of the patient's foot relative to the two upward force exerting means (2).

The hindfoot blocking support (17) is sensibly located along a perpendicular axis of the rails (15) in between the two upward force exerting means (2).

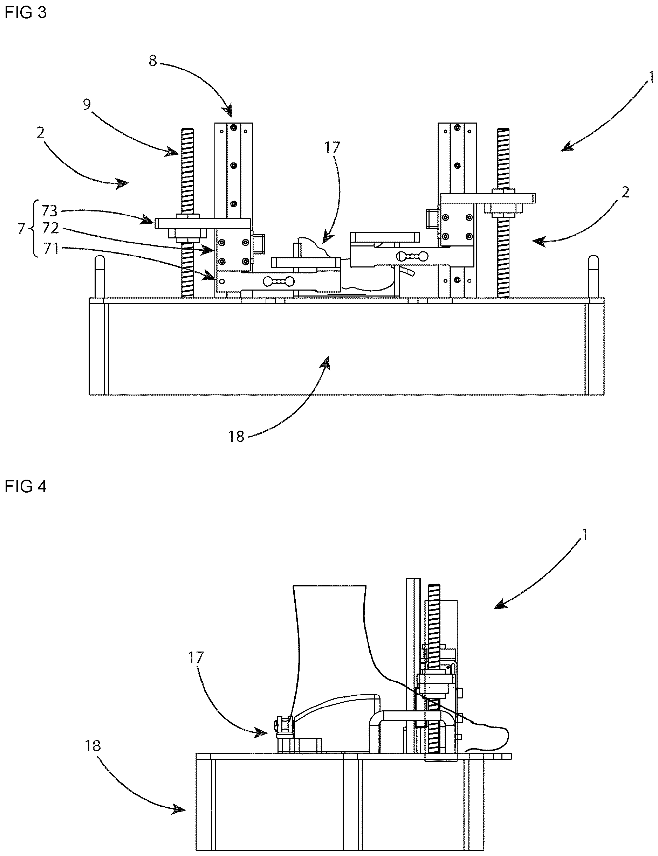

As illustrated in figures 3 and 4, the device (1) comprises a base housing (18) on which is located the hindfoot blocking support (17).

At least the actuator (6), the sliding platform (11) and the secondary actuator (13) are located within the base housing (18), while at least the linear guide (8) and the lead screw (9) emerged from the base housing (18).

As illustrated in figure 4, the device (1) is configured in such a way that it can adopt a neutral position where the two foot supports (10) showed on the top surface of the base housing (18), in order that the foot can adopt a neutral position on the base housing (18) while held by the hindfoot blocking support (17).

As illustrated in figure 1 in comparison with figure 2, the device (1) comprises protecting housing located around the different functioning parts, as the lead screw (9) and the linear guide (8) to protect the patient from moving mechanisms.

Figure 8 represents two curves showing measurements of the absolute displacement in millimeters (mm) of each actuator (6) as a function of the applied force in Newton (N) on respective force sensors.

In other words, the graph of figure 8 shows a measurement of the absolute displacement of a first actuator (6) as a function of the applied force on M1 metatarsal head, and the absolute displacement of a second actuator (6) as a function of the applied force on M2-M5 metatarsal heads.

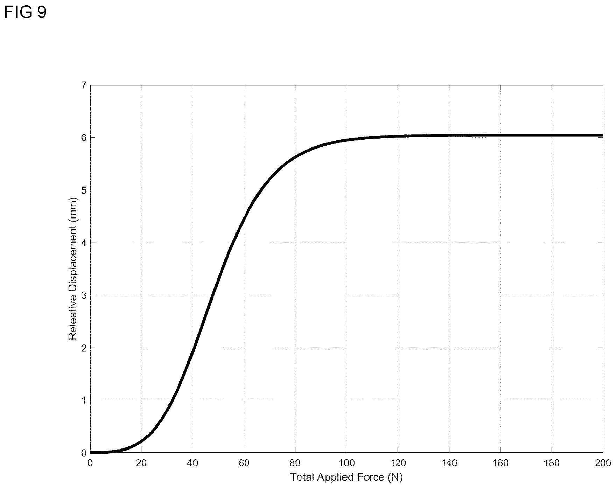

Figure 9 represents a graph showing the resulting relative displacement in millimeters of the two actuators (6) as a function of the sum of the applied forces in Newton (N).

In other words, the graph of figure 9 shows the difference of displacement between the M1 and M2-M5 metatarsal heads as a function of the global force applied to the forefoot.

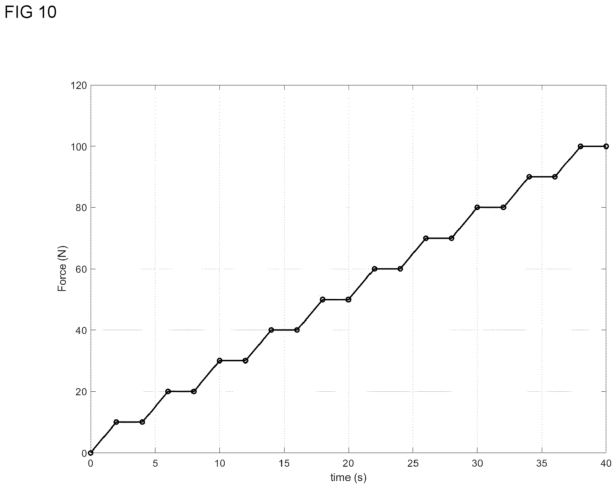

Figure 10 represents a graph showing the control of the applied force by an actuator (6) as a function of time. This control mode allows to gradually increase and maintain the applied force in level, and obtain measurement, not shown, for each level of the applied force, the response in displacement of the M1 and/or M2-M5 metatarsals. This control mode thus permits to obtain the gradual responses of the dorsal mobility of the first ray of the foot as a function of the level of applied force, reducing the influence of soft tissues and dynamic behavior of the foot, increasing the quality of the measurement and thus its reliability.

## Claims

1. Device (1) for measuring the dorsal mobility of the first ray of the foot comprising two independent upward force exerting means (2) adapted to exert a force on a part of a forefoot, each upward force exerting means (2) comprises a force sensor, wherein the device (1) comprises at least one displacement sensor (5), the device (1) is adapted to measure a displacement as a function of the applied force.

2. Device (1) of claim 1, wherein each force exerting means (2) comprises a displacement sensor (5).

3. Device (1) of claim 1 or 2, wherein the two upward force exerting means (2) are arranged in opposition to each other and are adapted to exert a force respectively on the internal and external part of the forefoot.

4. Device (1) of anyone of claims 1 to 3, wherein each upward force exerting means (1) comprises an output shaft (3) driven by an actuator (6).

5. Device (1) of claim 4, wherein the actuator (6) is an electronically controlled actuator.

6. Device (1) of claim 5, wherein the actuator (6) is a stepper motor associated with a digital stepper driver as a displacement sensor (5).

7. Device (1) of anyone of claims 4 to 6, wherein the output shaft (3) is perpendicularly connected to a support arm (7) mobile in translation along a linear guide (8) directed upwardly.

8. Device (1) of claim 7, wherein the support arm (7) is connected to a lead screw (9) coupled to the actuator (6), while the lead screw (9) is arranged parallel to the linear guide (8).

9. Device (1) of claim 8, wherein the support arm (7) comprises a first arm (71) connected to the output shaft (3), a linear carriage (72) mounted along the linear guide (8), and second arm (73) mounted along the lead screw (9).

10. Device (1) of anyone of claims 4 to 9, wherein the distal end of the output shaft (3) is linked to a forefoot support (10) adapted to support sensibly half of the lateral part of the forefoot.

11. Device (1) of claim 10, wherein the forefoot support (10) comprises a matrix array sensor.

12. Device (1) of anyone of claim 1 to 11, wherein the two independent upward force exerting means (2) are laterally mobile in translation to adapt to the width of a forefoot.

13. Device (1) of claim 12, wherein each upward force exerting means (2) are independently, laterally mobile in translation.

14. Device (1) of claim 12 or 13, wherein each upward force exerting means (2) is disposed on a sliding platform (11) driven by a secondary actuator (13).

15. Device (1) of anyone of claims 1 to 14, wherein it comprises a hindfoot blocking support (17).
